# EUROPEAN PATENT APPLICATION

(11) **EP 4 643 774 A1**
(43) Date of publication of application: **05.11.2025**
(21) Application number: 22969654.7
(22) Date of filing: 29.12.2022
(51) Int. Cl.: A61B 5/145

(54) **IMPLANT APPARATUS FOR CONTINUOUS BLOOD GLUCOSE MONITOR**

(30) Priority: 28.12.2022 CN 202211695874
(71) Applicant: Jiangsu Yuwell Poctech Biotechnology Co., Ltd., Zhenjiang, Jiangsu 212300 (CN); Zhejiang Poctech Co., Ltd., Huzhou, Zhejiang 313001 (CN); Jiangsu Yuekai Biotechnology Co., Ltd., Nanjing, Jiangsu 210018 (CN)
(72) Inventor: YANG, Kaihong, Zhenjiang, Jiangsu 212300 (CN); ZHANG, Yanan, Zhenjiang, Jiangsu 212300 (CN); ZHI, Jiajia, Zhenjiang, Jiangsu 212300 (CN); ZHOU, Hualong, Zhenjiang, Jiangsu 212300 (CN); PU, Jirui, Zhenjiang, Jiangsu 212300 (CN); CHEN, Feng, Zhenjiang, Jiangsu 212300 (CN); SUN, Bozhen, Zhenjiang, Jiangsu 212300 (CN); SHI, Yiqi, Zhenjiang, Jiangsu 212300 (CN); HUA, Hao, Zhenjiang, Jiangsu 212300 (CN)
(74) Representative: Nederlandsch Octrooibureau
(86) International application number: PCT/CN2022/143258
(87) International publication number: WO 2024/138505

(57) **Abstract**

Provided is an implant apparatus for a continuous glucose monitor. The implant apparatus includes a housing assembly and a driving assembly disposed in the housing assembly. The housing assembly includes an outer housing. The outer housing has an opening at an end of the outer housing. The driving assembly has a locked state in which the driving assembly is fixed relative to the outer housing and a triggered state in which the driving assembly is movable towards the opening. The implant apparatus further includes a trigger. The outer housing includes a guiding channel located at a side wall of the outer housing and extending towards the opening. The trigger is movable along the guiding channel towards the opening and has a first position and a second position relative to the outer housing, to enable the driving assembly to switch from the locked state to the triggered state. A triggering direction of the trigger is oriented towards the opening, while a direction of a force applied by fingers of a user when the user holds the outer housing is oriented inwards in a radial direction of the outer housing. Force application directions of the two actions are perpendicular to each other. As a result, a probability of the user accidentally triggering the trigger when holding the outer housing is reduced, which reduces a risk of accidental triggering of the implant apparatus, improving use experience.

## Description

### CROSS-REFERENCE TO RELATED APPLICATION

This application claims priority to Chinese patent application No. 202211695874.4, titled "IMPLANT APPARATUS FOR CONTINUOUS GLUCOSE MONITOR" and filed with China National Intellectual Property Administration on December 28, 2022, the entire contents of which are incorporated herein by reference.

### FIELD

The present disclosure belongs to the field of medical device technologies, and more particularly, to an implant apparatus for a continuous glucose monitor.

### BACKGROUND

A biosensor is an instrument that is sensitive to bioactive substances and converts a concentration of sensed bioactive substances into an electrical signal for a detection. A glucose sensor is a relatively common type of biosensor. Continuous Glucose Monitoring, CGM, is a technical means that continuously monitors a glucose concentration in an interstitial fluid under the skin through the glucose sensor to indirectly reflect a blood glucose level.

When using a CGM product, a user needs to place a housing of the CGM product on the skin and presses a trigger button. In this case, a puncture needle and a sensor pin inside the housing move towards and pierce the skin. An electrochemical reaction occurs between biological enzymes on the sensor and the interstitial fluid under the skin, and is converted into an electrical signal. The electrical signal is converted into blood glucose value readings for the user.

A trigger structure of a conventional CGM product is generally a button structure disposed at a side wall of the housing. When using the conventional CGM product, the user needs to hold the housing, and then press the button inwards to complete a trigger operation. However, for this trigger operation, a direction of a force applied by fingers of the user when the user holds the housing is the same a direction of a force applied by the trigger button, both of which are oriented towards an interior of the housing in a radial direction of the housing. Therefore, when the user holds the housing with a large grip force, especially when the user experiences fear during use, there is a large risk of accidental triggering. That is, an implant apparatus is triggered when the user accidentally presses the trigger button. Consequently, an implantation action and a needle withdrawal action of the puncture needle in the implant apparatus are completed before the user is fully prepared. However, the CGM products are often disposable, that is, the puncture needle cannot be reused after one implantation. Therefore, an accidental trigger operation directly causes the product to be unusable, greatly increasing use costs.

To address the above problems, some manufacturers have set up safety lock structures at the CGM products to reduce a probability of accidental triggering by the user. However, these locking structures are complicated, and require precise assembly with other parts of the products, which undoubtedly greatly increases manufacturing costs and assembly difficulty.

In addition, an arrangement of the safety lock requires the user to perform extra operations to unlock the safety lock before performing an implantation operation. However, the conventional CGM products have various unlocking methods, which is difficult for the user to get started and causes the user to take a long time to get familiar with use methods of different products, leading to high learning costs. As a result, complexity of use and difficulty of operation are increased, resulting in unsatisfactory use experience.

### SUMMARY

The present disclosure provides an implant apparatus for a continuous glucose monitor to solve at least one of the above-described technical problems.

Technical solutions adopted in the present disclosure are as follows.

An implant apparatus for a continuous glucose monitor is provided. The implant apparatus includes a housing assembly and a driving assembly disposed in the housing assembly. The housing assembly includes an outer housing. The outer housing has an opening at an end of the outer housing. The driving assembly has a locked state in which the driving assembly is fixed relative to the outer housing and a triggered state in which the driving assembly is movable towards the opening. The implant apparatus further includes a trigger. The outer housing includes a guiding channel located at a side wall of the outer housing and extending towards the opening. The trigger is movable towards the opening along the guiding channel and has a first position and a second position relative to the outer housing, to enable the driving assembly to switch from the locked state to the triggered state.

The trigger covers the guiding channel when the trigger is located at the first position or the second position.

The implant apparatus further includes a cap configured to block the opening and configured to engage with the trigger to restrict a movement of the trigger.

The trigger surrounds an outer periphery of the housing assembly.

The housing assembly is provided with an engagement wing, and the engagement wing is provided with a stop rib. The trigger includes a triggering portion extending into the outer housing through the guiding channel. The triggering portion is configured to press the engagement wing to displace the stop rib, to enable the driving assembly to switch from the locked state to the triggered state.

The engagement wing has an inclined engagement surface at an outer side of the engagement wing. The triggering portion is engaged with the inclined engagement surface to press the engagement wing to move towards an interior of the outer housing.

The engagement wing has a fixed end fixedly connected to the housing assembly, and a free end. The inclined engagement surface and the stop rib are disposed at the free end.

The driving assembly has an engagement rib. The driving assembly is in the locked state when the stop rib is in a stop engagement with the engagement rib. The driving assembly is in the triggered state when the stop rib is offset from the engagement rib.

The housing assembly further includes an inner housing disposed in the outer housing. The engagement wing is disposed at the inner housing.

The outer housing is provided with a snap portion at an inner wall of the outer housing. The inner housing is provided with an engagement portion at an outer wall of the inner housing, the snap portion being engaged with and fixed to the engagement portion.

The outer housing includes an upper housing and a lower housing assembled with each other. The upper housing has an upper slide channel. The lower housing has a lower slide channel, the upper slide channel being engaged with the lower slide channel to form the guiding channel.

The trigger is provided with a force application portion.

The implant apparatus further includes a puncture assembly fixed to the driving assembly. The driving assembly has a distal position and a proximal position. The driving assembly is configured to drive the puncture assembly to move relative to the housing assembly from the distal position to the proximal position. The driving assembly is provided with a limit portion configured to limit a position of the puncture assembly. The housing assembly is provided with an unlocking structure configured to release a positional limitation of the limit portion when the driving assembly is moved to the proximal position, to move the puncture assembly away from the opening relative to the driving assembly.

The implant apparatus further includes a connection base, a skin fixing base, and a sensor. The connection base is fixed to the driving assembly and has a mounting site inside the connection base, the mounting site being configured for mounting of the skin fixing base. The skin fixing base has a through hole. The sensor is at least partially received in the puncture assembly. The sensor has an end located inside the skin fixing base and another end extending downwards through the through hole. The puncture assembly passes through the through hole. The connection base is provided with a limit structure configured to at least restricting the puncture assembly from moving out of the through hole.

The implant apparatus further includes a cap configured to blocking the opening of the outer housing and cooperating with the limit structure to allow the limit structure to be linked with the cap.

The driving assembly has a slide channel configured to guide needle assistance and/or needle withdrawal of the puncture assembly. The limit portion includes a stop protrusion disposed at the slide channel. The puncture assembly has an abutment wing configured to abut with the stop protrusion to restrict the puncture assembly from moving away from the opening.

The unlocking structure includes an unlocking rib disposed at the housing assembly and configured to abut against the abutment wing to move the abutment wing and disengage the abutment wing from the stop protrusion.

Since the above-described technical solutions are adopted, the present disclosure can provide the following advantageous effects.
1. In the present disclosure, the outer housing is provided with the guiding channel, and the guiding channel is engaged with the trigger, in such a manner that the trigger can move towards the opening to trigger the implant apparatus. Unlike a manner of pressing a trigger button, the present disclosure adopts a method where a triggering direction of the trigger is oriented towards the opening, i.e., in an axis direction of the outer housing, while a direction of a force applied by fingers of a user when the user holds the outer housing is oriented inwards in a radial direction of the outer housing. Force application directions of the two actions are perpendicular to each other. As a result, a probability of the user accidentally triggering the trigger when holding the outer housing is reduced, which reduces a risk of accidental triggering of the implant apparatus, and ensures that the implant apparatus can be effectively triggered and implanted, improving use experience.

In addition, since a movement direction of the trigger is oriented towards the opening, prevention of accidental triggering is further improved. Triggering can only be achieved when a relative movement occurs between the outer housing and the trigger. Specifically, when the opening of the outer housing abuts with the skin of a patient, the outer housing is stopped by the skin, in such a manner that the outer housing cannot move towards the skin. In this case, the user can better operate the trigger to move the trigger relative to the outer housing, completing a trigger operation. However, when an opening end of the outer housing fails to abut with the skin, the user pushing the trigger causes the outer housing to move with the trigger. In this way, the user cannot apply a force to the trigger, and no relative movement occurs between the trigger and the outer housing, in such a manner that the implant apparatus cannot be triggered. Therefore, by designing the movement direction of the trigger to be the same as an implantation direction of the driving assembly, the trigger can obtain a certain level of prevention of accidental triggering without additionally setting up a safety lock, which simplifies a structure of the implant apparatus, saving costs. In addition, operation steps of the user when using the implant apparatus are simplified, which improves the use experience.

2. As a preferred embodiment of the present disclosure, the implant apparatus further includes the cap configured to block the opening and configured to engage with the trigger to restrict the movement of the trigger. When the implant apparatus leaves a factory, the opening end of the outer housing is provided with the cap configured to block the opening of the outer housing. Members of the implant apparatus are all located in a cavity formed by the cap and the outer housing. The cap and the outer housing define a sterile environment which is isolated from an external environment, ensuring internal cleanliness. In addition, the cap can also restrict the movement of the trigger, in such a manner that the trigger cannot move towards the opening, that is, cannot be triggered. Therefore, the prevention of the accidental triggering of the implant apparatus is further improved, providing multiple protection for the trigger, and ensuring that the trigger is triggered correctly. Before using the implant apparatus, the user removes the cap to expose the opening of the outer housing. At this point, the opening can be abutted with a skin surface for an implantation operation. In addition, a removal of the cap eliminates a restriction on the trigger, enabling the trigger to be triggered normally to move towards the opening.

3. As a preferred embodiment of the present disclosure, the housing assembly is provided the engagement wing. The engagement wing is provided with the stop rib. The trigger includes the triggering portion extending into the outer housing through the guiding channel. The triggering portion is configured to press the engagement wing to displace the stop rib, to enable the driving assembly to switch from the locked state to the triggered state. When the trigger is located at the first position, the engagement wing is in the stop engagement with the driving assembly to restrict the driving assembly to an original position, which prevents the driving assembly from moving towards the opening for an implantation action. When the user moves the trigger to the second position, the triggering portion presses the engagement wing, and the engagement wing is deformed when being pressed, which drives the stop rib to move for avoiding the driving assembly. In this case, the stop rib no longer stops the driving assembly, and thus the driving assembly can perform the implantation action to complete the implantation.

4. As a preferred embodiment of the present disclosure, the implant apparatus further includes the puncture assembly fixed to the driving assembly. The driving assembly has the distal position and the proximal position. The driving assembly is configured to drive the puncture assembly to move relative to the housing assembly from the distal position to the proximal position. The driving assembly is provided with the limit portion configured to restrict the position of the puncture assembly. The housing assembly is provided with the unlocking structure. The unlocking structure is configured to release the positional limitation of the limit portion when the driving assembly is moved to the proximal position, to move the puncture assembly away from the opening relative to the driving assembly. When the user triggers the implantation operation, the driving assembly and the puncture assembly move synchronously from the distal position to the proximal position together. At this point, the puncture assembly punctures the skin of the patient to implant a sensor needle inside the skin fixing base under the skin of the patient. The position of the puncture assembly is restricted by the limit portion, which enables the puncture assembly to move synchronously with the driving assembly. When the implantation is completed, the puncture assembly is triggered by the unlocking structure and releases a restriction of the limit portion, enabling the puncture assembly to move relative to the driving assembly. In this case, the driving assembly remains at the proximal position, while the puncture assembly retracts alone to complete a needle withdrawal operation. The unlocking structure is located at the proximal position and at a movement path of the puncture assembly. Therefore, when the driving assembly carries the puncture assembly to move to the proximal position together, the unlocking structure can automatically release the puncture assembly without requiring the user to perform the needle withdrawal operation. Consequently, the implantation and needle withdrawal are automatically and consecutively completed in a single-step operation, which simplifies operation steps of the user and reduces operation difficulty, improving the use experience.

5. As a preferred embodiment of the present disclosure, the implant apparatus further includes the connection base, the skin fixing base, and the sensor. The connection base is fixed to the driving assembly and has the mounting site inside the connection base. The mounting site is configured for mounting of the skin fixing base. The skin fixing base has the through hole. The sensor is at least partially received in the puncture assembly. The sensor has the end located inside the skin fixing base and the other end extending downwards through the through hole. The puncture assembly passes through the through hole. The connection base is provided with the limit structure configured to at least restrict the puncture assembly from moving out of the through hole. The limit structure can restrict the position of the puncture assembly, in such a manner that the puncture assembly remains firmly connected to the implant apparatus, which reduces a risk of detachment between the puncture assembly and the implant apparatus. In addition, the limit structure can restrict a movement of the puncture assembly to realize the prevention of the accidental triggering of the puncture assembly. Under a restriction of a limit assembly, the puncture assembly cannot move and thus cannot carry out the implantation action and the needle withdrawal action, which improves operation reliability of the implant apparatus and saves the costs.

### BRIEF DESCRIPTION OF THE DRAWINGS

The accompanying drawings described here are used to provide a further understanding of the present disclosure and constitute a part of the present disclosure. Exemplary embodiments of the present disclosure and description thereof are used to explain the present disclosure, and do not constitute an improper limitation of the present disclosure. In the accompanying drawings:
FIG. 1 is a schematic structural view of an implant apparatus according to an embodiment of the present disclosure.
FIG. 2 is a cross-sectional view of the implant apparatus in FIG. 1, in which a puncture assembly and a skin fixing base are not illustrated.
FIG. 3 is a cross-sectional view of the implant apparatus in FIG. 2, viewed from another perspective.
FIG. 4 is a schematic structural view of an outer housing according to an embodiment of the present disclosure.
FIG. 5 is a schematic view of an internal structure of the outer housing in FIG. 4.
FIG. 6 is a schematic structural view of a trigger according to an embodiment of the present disclosure.
FIG. 7 is a schematic structural view of an inner housing according to an embodiment of the present disclosure.
FIG. 8 is a cross-sectional view of the inner housing in FIG. 7.
FIG. 9 is a schematic structural view of a driving assembly according to an embodiment of the present disclosure.
FIG. 10 is a front view of the driving assembly in FIG. 9.
FIG. 11 is a cross-sectional view of the driving assembly in FIG. 9.
FIG. 12 is a schematic structural view of a needle base according to an embodiment of the present disclosure.
FIG. 13 is a cross-sectional view of the needle base in FIG. 12.
FIG. 14 is a schematic structural view of an implant apparatus according to another embodiment of the present disclosure.
FIG. 15 is a schematic structural view of a partial region inside an implant apparatus according to an embodiment of the present disclosure.
FIG. 16 is a cross-sectional view of the implant apparatus in FIG. 15.
FIG. 17 is a schematic structural view of a needle body according to an embodiment of the present disclosure.
FIG. 18 is a schematic view of an internal structure of an implant apparatus according to an embodiment of the present disclosure.
FIG. 19 is a schematic structural view of a skin fixing base according to an embodiment of the present disclosure.
FIG. 20 is a schematic view of an internal structure of the skin fixing base in FIG. 19.

1 outer housing; 11 guiding channel; 12 snap protrusion; 13 limit snap;
2 driving assembly; 21 tension spring; 22 first positioning post; 23 propelling spring; 24 engagement rib; 25 slide channel; 26 stop protrusion; 261 guide channel; 27 snap opening;
3 trigger; 31 force application portion; 32 triggering portion;
4 inner housing; 41 second positioning post; 42 engagement wing; 421 inclined engagement surface; 422 stop rib; 43 snap slot; 44 unlocking rib;
5 puncture assembly; 51 needle body; 511 engagement groove; 52 needle base; 521 guide cantilever; 522 abutment wing; 523 guide rib; 524 engagement claw;
6 cap; 61 sealed cavity;
7 connection base; 71 limit structure; 711 limit hole; 72 snap rib;
8 skin fixing base; 81 through hole; 82 sensor.

### DETAILED DESCRIPTION

To explain an overall concept of the present disclosure more clearly, detailed description is made below by way of examples with reference to the accompanying drawings.

In the following description, many specific details are provided to facilitate full understanding of the present disclosure. However, the present disclosure can further be implemented in many other ways than those described herein. Therefore, the scope of the present disclosure is not limited by specific embodiments disclosed below.

In addition, in the description of the present disclosure, it should be understood that, the orientation or the position indicated by terms such as "top", "bottom", "inner", "outer", "axial", "radial", and "circumferential" should be construed to refer to the orientation and the position as shown in the drawings, and is only for the convenience of describing the present disclosure and simplifying the description, rather than indicating or implying that the pointed device or element must have a specific orientation, or be constructed and operated in a specific orientation, and therefore cannot be understood as a limitation of the present disclosure.

In the present disclosure, unless otherwise clearly specified and limited, terms such as "install", "connect", "connect to", "fix", and the like should be understood in a broad sense. For example, it may be a fixed connection or a detachable connection or connection as one piece; mechanical connection or electrical connection or communication; direct connection or indirect connection through an intermediate; internal communication of two components or the interaction relationship between two components. For those of ordinary skill in the art, the specific meaning of the above-mentioned terms in the present disclosure can be understood according to specific circumstances.

In the present disclosure, unless expressly stipulated and defined otherwise, the first feature "on" or "under" the second feature may mean that the first feature is in direct contact with the second feature, or the first and second features are in indirect contact through an intermediate. Reference throughout this specification to "implementation", "embodiment", "an embodiment", "example", or "a specific example" means that a particular feature, structure, material, or characteristic described in connection with the embodiment or example is included in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples.

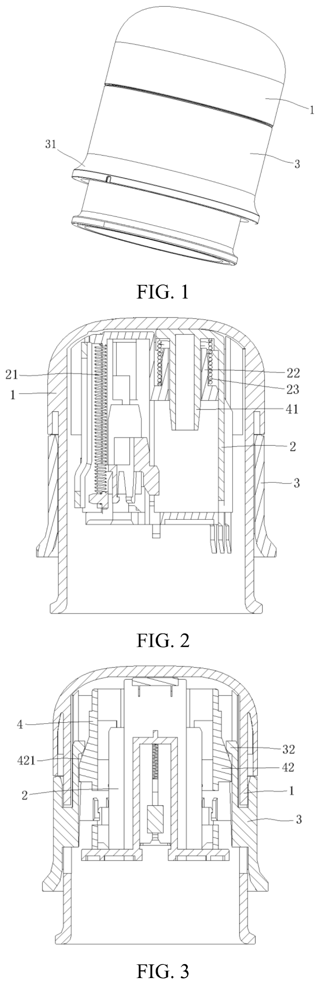

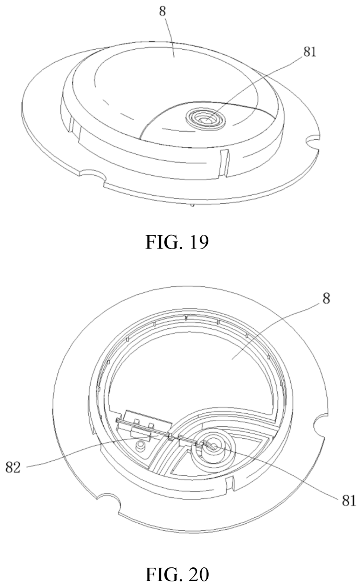

As illustrated in FIG. 1 to FIG. 20, an implant apparatus for a continuous glucose monitor is provided. The implant apparatus includes a housing assembly and a driving assembly 2 disposed in the housing assembly. The housing assembly includes an outer housing 1. The outer housing 1 has an opening at an end of the outer housing 1. The driving assembly 2 has a locked state in which the driving assembly 2 is fixed relative to the outer housing 1 and a triggered state in which the driving assembly 2 is movable towards the opening. The implant apparatus further includes a trigger 3. The outer housing 1 has a guiding channel 11 at a side wall of the outer housing 1. The guiding channel 11 extends towards the opening. The trigger 3 is movable towards the opening along the guiding channel 11 and has a first position and a second position relative to the outer housing 1, to enable the driving assembly 2 to switch from the locked state to the triggered state.

Preferably, as illustrated in FIG. 1, the outer housing 1 expands outwards at the opening to increase a contact area between an end portion of the outer housing 1 and skin, which reduces a pressure exerted by the outer housing 1 on the skin, improving use comfort.

In the present disclosure, the outer housing 1 is provided with the guiding channel 11, and the guiding channel 11 is engaged with the trigger 3, in such a manner that the trigger 3 can move towards the opening to trigger the implant apparatus. Unlike a manner of pressing a trigger button, the present disclosure adopts a method where a triggering direction of the trigger 3 is oriented towards the opening, i.e., in an axis direction of the outer housing 1, while a direction of a force applied by fingers of a user when the user holds the outer housing 1 is oriented inwards in a radial direction of the outer housing 1. Force application directions of the two actions are perpendicular to each other. As a result, a probability of the user accidentally triggering the trigger 3 when holding the outer housing 1 is reduced, which reduces a risk of accidental triggering of the implant apparatus, and ensures that the implant apparatus can be effectively triggered and implanted, improving use experience.

In addition, since a movement direction of the trigger 3 is oriented towards the opening, prevention of accidental triggering is further improved. Triggering can only be achieved when a relative movement occurs between the outer housing 1 and the trigger 3.

Specifically, when the opening of the outer housing 1 abuts with the skin of a patient, the outer housing 1 is stopped by the skin, in such a manner that the outer housing 1 cannot move towards the skin. In this case, the user can better operate the trigger 3 to move the trigger 3 relative to the outer housing 1, completing a trigger operation. However, when an opening end of the outer housing 1 fails to abut with the skin, the user pushing the trigger 3 causes the outer housing 1 to move with the trigger 3. In this way, the user cannot apply a force to the trigger 3, and no relative movement occurs between the trigger 3 and the outer housing 1, in such a manner that the implant apparatus cannot be triggered.

Therefore, by designing the movement direction of the trigger 3 to be the same as an implantation direction of the driving assembly 2, the trigger 3 can obtain a certain level of prevention of accidental triggering without additionally setting up a safety lock, which simplifies a structure of the implant apparatus, saving costs. In addition, operation steps of the user when using the implant apparatus are simplified, which improves the use experience.

It should be noted that the present disclosure does not specifically limit an extension direction of the guiding channel 11. The extension direction of the guiding channel may be set according to one of the following embodiments.

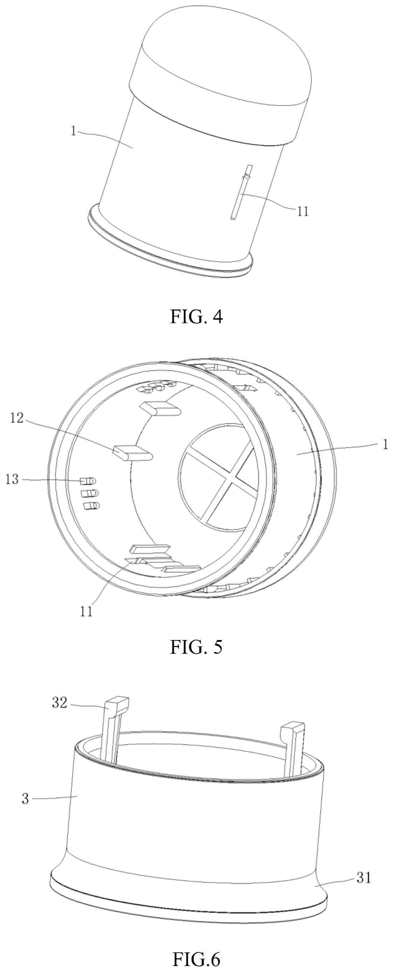

First embodiment: in this embodiment, as illustrated in FIG. 4, the guiding channel 11 extends in an axial direction of the outer housing 1 to enable the trigger 3 to move up and down in the axial direction of the outer housing 1. During an operation, the user needs to pinch the trigger 3 and press the trigger 3 downwards.

Second embodiment: in this embodiment, the guiding channel 11 is curved and extends from an upper end of the outer housing 1 to a lower end of the outer housing 1. For example, the guiding channel 11 extends in a spiral shape to enable the trigger 3 to rotate and move towards the opening. When the user operates the trigger 3, the trigger 3 needs to be rotated, in such a manner that the trigger 3 rotates about an axis of the outer housing 1 and moves towards the opening under guidance of the spiral slide channel.

As a preferred embodiment of the present disclosure, the trigger 3 covers the guiding channel 11 when the trigger 3 is located at the first position or the second position. No matter which position the trigger is located, the guiding channel 11 is covered. On the one hand, an appearance defect caused by exposure of the guiding channel 11 is avoided to avoid an influence on an overall appearance of the implant apparatus. On the other hand, the trigger 3 can also block external impurities such as dust from entering the outer housing 1 through the guiding channel 11, ensuring cleanliness inside the implant apparatus.

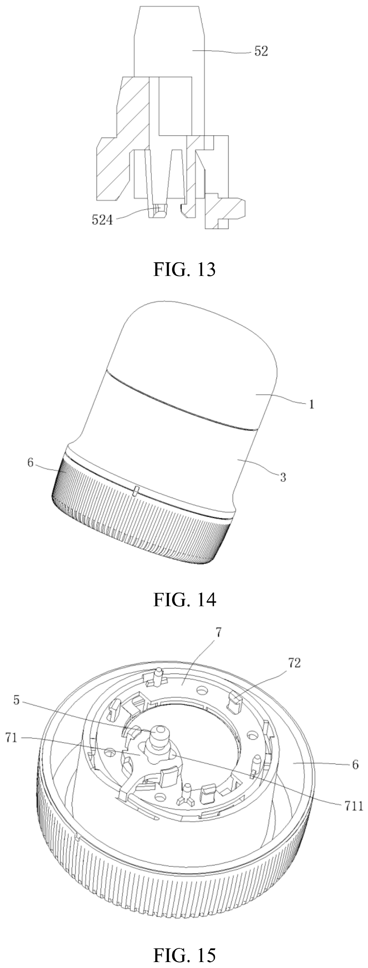

Preferably, as illustrated in FIG. 14, the implant apparatus further includes a cap 6 configured to block the opening and configured to engage with the trigger 3 to restrict a movement of the trigger 3.

When the implant apparatus leaves a factory, the opening end of the outer housing 1 is provided with the cap 6 configured to block the opening of the outer housing 1. Members of the implant apparatus are all located in a cavity formed by the cap 6 and the outer housing 1. The cap 6 and the outer housing 1 define a sterile environment which is isolated from an external environment, ensuring internal cleanliness.

In addition, the cap 6 can also restrict the movement of the trigger 3, in such a manner that the trigger 3 cannot move towards the opening, that is, cannot be triggered. Therefore, the prevention of the accidental triggering of the implant apparatus is further improved, providing multiple protection for the trigger 3, and ensuring that the trigger 3 is triggered correctly. Before using the implant apparatus, the user removes the cap 6 to expose the opening of the outer housing 1. At this point, the opening can be abutted with a skin surface for an implantation operation. In addition, a removal of the cap 6 eliminates a restriction on the trigger 3, enabling the trigger 3 to be triggered normally to move towards the opening.

A disassembly method of the cap 6 is not limited in the present disclosure. The cap 6 may be separated and removed from the outer housing 1 through a rotational movement, or the cap 6 may be removed through applying a force along the axis of the outer housing 1 to pull out the cap 6, or the like. The present disclosure is not limited in this regard.

In an embodiment, as illustrated in FIG. 1, the trigger 3 surrounds an outer periphery of the housing assembly.

The trigger 3 surrounding the housing assembly improves appearance integrity of the implant apparatus, enhances aesthetics, and facilitates use of the user. During an operation, the user can hold the trigger 3 with ring-like grip, which improves operational feel.

It should be understood that the trigger 3 surrounding the housing assembly further allows the user to hold the housing assembly with his or her fingers positioned outside the trigger 3. In this way, when the user has not pressed the opening against the skin, a great gripping force is required to hold the implant apparatus. At this point, the trigger 3 is slightly deformed inwards under an action of the gripping force and therefore abuts with the housing assembly, which increases a friction between the trigger 3 and the housing assembly, reducing a possibility of a relative movement between the trigger 3 and the housing assembly. Therefore, it becomes more difficult for the user to trigger the trigger 3, improving the prevention of the accidental triggering.

Of course, the trigger 3 may also have other structures. For example, the triggers 3 are symmetrically arranged at two sides of the housing assembly. When using the implant apparatus, the user can place the thumb on the trigger 3 at one side and the other four fingers on the trigger 3 at another side, applying forces simultaneously to press the trigger 3 at the two sides downwards to achieve the triggering.

As a preferred embodiment of the present disclosure, as illustrated in FIG. 3, FIG. 7, and FIG. 8, the housing assembly is provided with an engagement wing 42. The engagement wing 42 is provided with a stop rib 422. The trigger 3 includes a triggering portion 32 extending into the outer housing 1 through the guiding channel 11. The triggering portion 32 is configured to press the engagement wing 42 to displace the stop rib 422, to enable the driving assembly 2 to switch from the locked state to the triggered state.

Further, as illustrated in FIG. 3, FIG. 9, and FIG. 10, the driving assembly 2 has an engagement rib 24. The driving assembly 2 is in the locked state when the stop rib 422 is in a stop engagement with the engagement rib 24. The driving assembly 2 is in the triggered state when the stop rib 422 is offset from the engagement rib 24.

When the trigger 3 is located at the first position, the engagement wing 42 is in the stop engagement with the driving assembly 2 to restrict the driving assembly 2 to an original position, which prevents the driving assembly from moving towards the opening for an implantation action. When the user moves the trigger 3 to the second position, the trigger 3 presses the engagement wing 42, and the engagement wing 42 is deformed when pressed, which drives the stop rib 422 to move for avoiding the driving assembly 2. In this case, the stop rib 422 no longer stops the driving assembly 2, and thus the driving assembly 2 can perform the implantation action to complete the implantation.

It should be understood that, as illustrated in FIG. 3, the engagement wing 42 is located below the triggering portion 32 and at a movement path of the triggering portion 32. When the user presses the trigger 3 downwards, the triggering portion 32 moves downwards accordingly, and comes into contact with the engagement wing 42 to force the engagement wing 42 to move.

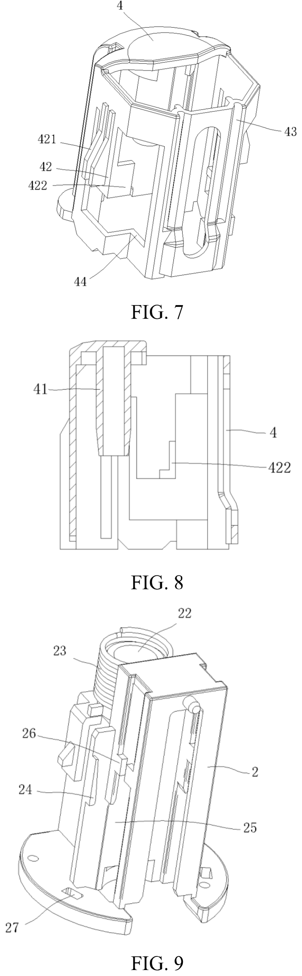

Further, as illustrated in FIG. 3 and FIG. 7, the engagement wing 42 has an inclined engagement surface 421 at an outer side of the engagement wing 42. The triggering portion 32 is engaged with the inclined engagement surface 421 to force the engagement wing 42 to move towards the interior of the outer housing 1.

With the inclined engagement surface 421, contact between the triggering portion 32 and the engagement wing 42 is softer and smoother, which not only ensures effective triggering of the engagement wing 42 by the triggering portion 32, but also improves the operational feel of the user and reduces a resistance during an operation, improving the use experience.

Preferably, as illustrated in FIG. 6, the triggering portion 32 is also provided with an inclined surface structure at a side of the triggering portion 32 facing towards the engagement wing 42, in such a manner that the triggering portion 32 and the engagement wing 42 are in contact and compress with each other along an inclined surface, further enhancing smoothness of an engagement.

As illustrated in FIG. 3, the engagement wings 42 are disposed at two sides of the driving assembly 2. That is, each of the stop ribs 422 at the two sides can form stop with the driving assembly 2 to improve stability of the driving assembly 2. Similarly, the triggering portions 32 are correspondingly arranged at the two sides. Under a movement of the trigger 3, the triggering portions 32 at the two sides simultaneously press the engagement wings 42 at the two sides, in such a manner that the two sides of the driving assembly 2 can be unlocked simultaneously.

The engagement wing 42 has a fixed end fixedly connected to the housing assembly, and a free end. The inclined engagement surface 421 and the stop rib 422 are both disposed at the free end.

The engagement wing 42 has a cantilever structure with a fixed end and a free end. Therefore, the free end of the engagement wing 42 can pivot around the fixed end under a press, in such a manner that the engagement wing 42 has satisfactory resilient deformation performance. The inclined engagement surface 421 and the stop rib 422 are both disposed at the free end. Therefore, the engagement wing 42 can undergo a large resilient deformation when being pressed, in such a manner that a response of the engagement wing 42 is more sensitive, realizing reliable unlocking and triggering.

In a preferred embodiment, as illustrated in FIG. 3 and FIG. 7, the housing assembly further includes an inner housing 4 disposed in the outer housing 1. The engagement wing 42 is disposed at the inner housing 4.

Further, the outer housing 1 is provided with a snap portion at an inner wall of the outer housing 1. The inner housing 4 is provided with an engagement portion at an outer wall of the inner housing 4. The snap portion is engaged with and fixed to the engagement portion.

Specifically, as illustrated in FIG. 5 and FIG. 7, the snap portion includes a snap protrusion 12. The engagement portion is a snap slot 43. The outer housing 1 and the inner housing 4 are fixed through an engagement between the snap protrusion 12 and the snap slot 43. Further, as illustrated in FIG. 5, the snap portion further includes a limit snap 13 disposed at the inner wall of the outer housing 1, and the limit snap 13 is engaged with the outer wall of the inner housing 4, in such a manner that a more stable connection is realized between the outer housing 1 and the inner housing 4. Of course, the outer housing 1 and the inner housing 4 can be fixed by other means, which is not specifically limited here.

In a preferred embodiment, the outer housing 1 includes an upper housing and a lower housing assembled with each other. The upper housing has an upper slide channel. The lower housing has a lower slide channel. The upper slide channel is engaged with the lower slide channel to form the guiding channel 11.

Since the upper slide channel is engaged with the lower slide channel to form the guiding channel 11, an engagement with the trigger 3 is facilitated. During an assembly, an engagement slide block of the trigger 3 may be put into the guiding channel 11, and then the upper housing is aligned with and fixed to the lower housing to enclose the guiding channel 11.

Preferably, as illustrated in FIG. 6, the trigger 3 is provided with a force application portion 31.

Specifically, as illustrated in FIG. 6, the trigger 3 has an outwardly expanded edge at an end of the trigger 3 facing towards the opening. In this way, the user can press the trigger 3 using the outwardly expanded edge, which facilitates the user to apply a force and reduces a possibility that the trigger 3 cannot be effectively operated due to slippage between the hand of the user and the trigger 3.

In a preferred embodiment of the present disclosure, as illustrated in FIG. 9, FIG. 15, and FIG. 18, the implant apparatus further includes a puncture assembly 5 fixed to the driving assembly 2. The driving assembly 2 has a distal position and a proximal position and is configured to drive the puncture assembly 5 to move relative to the housing assembly from the distal position to the proximal position. The driving assembly 2 is provided with a limit portion configured to limit a position of the puncture assembly 5. The housing assembly is provided with an unlocking structure configured to release a positional limitation of the limit portion when the driving assembly 2 is moved to the proximal position, to move the puncture assembly 5 away from the opening relative to the driving assembly 2.

When the user triggers the implantation operation, the driving assembly 2 and the puncture assembly 5 move synchronously from the distal position to the proximal position together. At this point, the puncture assembly 5 punctures the skin of the patient to implant a sensor needle inside a skin fixing base 8 under the skin of the patient. The position of the puncture assembly 5 is restricted by the limit portion, which enables the puncture assembly 5 to move synchronously with the driving assembly 2. When the implantation is completed, the puncture assembly 5 is triggered by the unlocking structure and releases a restriction of the limit portion, enabling the puncture assembly 5 to move relative to the driving assembly 2. In this case, the driving assembly 2 remains at the proximal position, while the puncture assembly 5 retracts alone to complete a needle withdrawal operation. The unlocking structure is located at the proximal position and at a movement path of the puncture assembly 5. Therefore, when the driving assembly 2 carries the puncture assembly 5 to move to the proximal position together, the unlocking structure can automatically release the puncture assembly 5 without requiring the user to perform the needle withdrawal operation. Consequently, the implantation and needle withdrawal are automatically and consecutively completed in a single-step operation, which simplifies operation steps of the user and reduces operation difficulty, improving the use experience.

It should be understood that, during use, the opening of the outer housing 1 faces towards the skin. Therefore, the distal position refers to a side away from the skin, and the proximal position refers to a side close to the skin. When the driving assembly 2 and the puncture assembly 5 move from the distal position to the proximal position, a needle assistance (implantation) action is performed. When the puncture assembly 5 moves from the proximal position to the distal position, a needle withdrawal action is performed.

Further, as illustrated in FIG. 9, FIG. 11, and FIG. 12, the driving assembly 2 has a slide channel 25 configured to guide needle assistance and/or needle withdrawal of the puncture assembly 5. The limit portion includes a stop protrusion 26 disposed at the slide channel 25. The puncture assembly 5 has an abutment wing 522 configured to abut with the stop protrusion 26 to restrict the puncture assembly 5 from moving away from the opening.

The slide channel 25 can guide a movement of the puncture assembly 5, and extends in a direction of needle assistance and needle withdrawal. In this way, a relative movement between the puncture assembly 5 and the driving assembly 2 is restricted to this direction only, improving reliability in an implantation and needle withdrawal process. The stop protrusion 26 is disposed at the slide channel 25. The abutment wing 522 of the puncture assembly 5 is stopped with the stop protrusion 26 to restrict the puncture assembly 5 at the proximal position. When the unlocking structure presses the abutment wing 522 to make the abutment wing 522 offset from the stop protrusion 26, the abutment wing 522 is no longer stopped by the stop protrusion 26, in such a manner that the puncture assembly 5 can move towards a distal end alone.

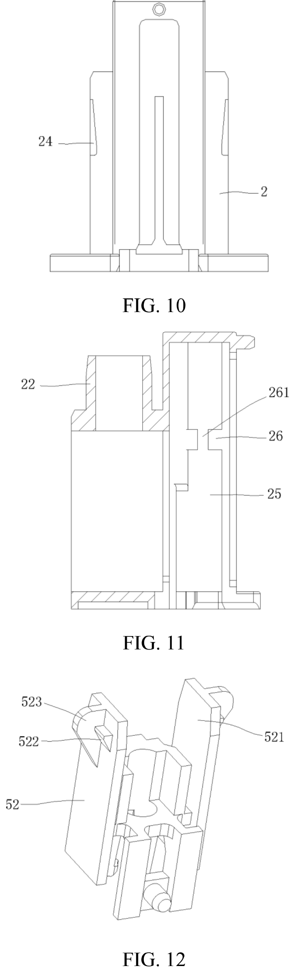

Further, as illustrated in FIG. 11, the stop protrusion 26 divides the slide channel 25 into two segments. An upper segment of the slide channel 25 forms a needle withdrawal stroke of the puncture assembly 5.

Furthermore, as illustrated in FIG. 11, two stop protrusions 26 are provided and arranged at two sides of the slide channel 25. A guide channel 261 is formed between the two stop protrusions 26. The abutment wing 522 is further provided with a guide rib 523 located in the guide channel 261 and movable along the guide channel 261.

A shape of the guide rib 523 is adapted to a shape of the guide channel 261. In this way, after the guide rib 523 and the guide channel 261 are fit with each other, the movement of the puncture assembly 5 can be further guided, improving reliability of a needle withdrawal movement of the puncture assembly 5.

Specifically, as illustrated in FIG. 2, FIG. 12, FIG. 17, and FIG. 18, the puncture assembly 5 includes a needle body 51, a needle base 52, a tension spring 21, and a guide cantilever 521 disposed at the needle base 52 and cooperating with the slide channel 25. When the puncture assembly 5 moves to the proximal position, the unlocking structure presses a free end of the guide cantilever 521, which causes the guide cantilever 521 to be deformed. In this way, the abutment wing 522 and the stop protrusion 26 are offset from each other, in such a manner that the abutment wing 522 is no longer stopped by the stop protrusion 26, triggering the needle withdrawal movement.

As illustrated in FIG. 13, the needle base 52 includes an engagement claw 524, and the needle body 51 has an engagement groove 511. The engagement claw 524 and the engagement groove 511 are engaged with each other to fix the needle base 52 with the needle body 51.

The tension spring 21 has an end fixed to the driving assembly 2 and another end fixed to the needle base 52. In an initial state, the tension spring 21 is stretched. When the driving assembly 2 and the puncture assembly 5 move together to the proximal position to complete the implantation, the limit portion is triggered by the unlocking structure. In this way, the puncture assembly 5 is no longer stopped by the limit portion, and the needle base 52 moves towards the distal end under a pulling force of the tension spring 21 to complete the needle withdrawal.

More specifically, as illustrated in FIG. 2, FIG. 9, and FIG. 18, the implant apparatus further includes a propelling spring 23. The propelling spring 23 has an end acting on the housing assembly and another end acting on the driving assembly 2. In an initial state, the propelling spring 23 is pressed. After the user performs the trigger operation, the driving assembly 2 carries the puncture assembly 5 to move towards the proximal position under an elastic force of the propelling spring 23 to complete the implantation.

As illustrated in FIG. 8 and FIG. 9, the driving assembly 2 is provided with a first positioning post 22. The housing assembly is provided with a second positioning post 41. The first positioning post 22 and the second positioning post 41 are arranged around each other. The propelling spring 23 is arranged around the first positioning post 22 and the second positioning post 41, or between the first positioning post 22 and the second positioning post 41.

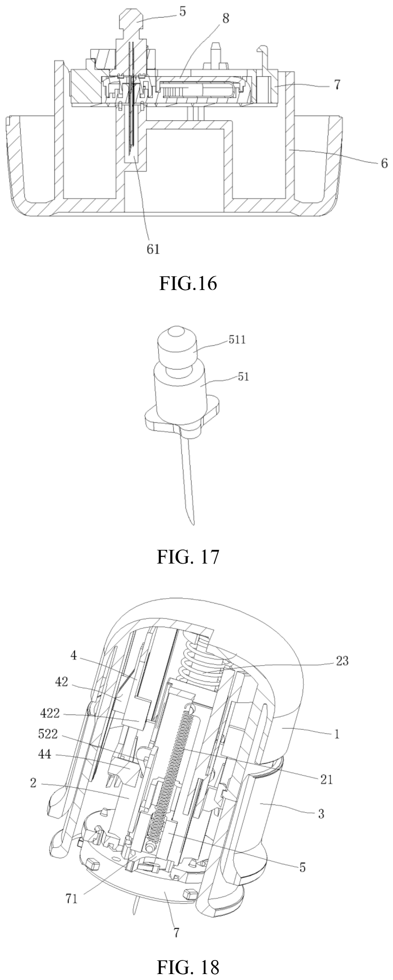

Preferably, as illustrated in FIG. 7 and FIG. 18, the unlocking structure includes an unlocking rib 44 disposed at the housing assembly and configured to abut against the abutment wing 522 to move the abutment wing 522 and disengage the abutment wing 522 from the stop protrusion 26.

The unlocking rib 44 is located at the proximal position. When the driving assembly 2 and the puncture assembly 5 move to the proximal position, the abutment wing 522 collides with the unlocking rib 44 under kinetic energy of the driving assembly 2, and the unlocking rib 44 presses the abutment wing 522 to disengage the abutment wing 522 from the stop protrusion to complete unlocking.

In a preferred embodiment of the present disclosure, as illustrated in FIG. 15, FIG. 16, FIG. 18, FIG. 19, and FIG. 20, the implant apparatus further includes a connection base 7, the skin fixing base 8, and a sensor 82. The connection base 7 is fixed to the driving assembly 2 and has a mounting site inside the connection base 7. The mounting site is configured for mounting of the skin fixing base 8. The skin fixing base 8 has a through hole 81. The sensor 82 is at least partially received in the puncture assembly 5. The sensor 82 has an end located inside the skin fixing base 8 and another end extending downwards through the through hole 81. The puncture assembly 5 passes through the through hole 81. The connection base 7 is provided with a limit structure 71 configured to at least restrict the puncture assembly 5 from moving out of the through hole 81.

Preferably, the connection base 7 is in a snap-fit with and fixed to the driving assembly 2. Specifically, as illustrated in FIG. 9 and FIG.15, the connection base 7 is provided with a snap rib 72. The driving assembly 2 correspondingly has a snap opening 27. The snap rib 72 passes through the snap opening 27 to be in a snap-fit with and fixed to the driving assembly 2.

The limit structure 71 can restrict the position of the puncture assembly 5, in such a manner that the puncture assembly 5 remains firmly connected to the implant apparatus, which reduces a risk of detachment between the puncture assembly 5 and the implant apparatus. In addition, the limit structure 7 can restrict the movement of the puncture assembly 5 to realize the prevention of the accidental triggering of the puncture assembly 5. Under a restriction of the limit structure 71, the puncture assembly 5 cannot move and thus cannot carry out the implantation action and the needle withdrawal action, which improves operation reliability of the implant apparatus and saves the costs.

Further, as illustrated in FIG. 15, the limit structure 71 has a limit hole 711 in which at least part of the puncture assembly 5 is accommodated. The limit hole 711 is provided with a first stop structure. The puncture assembly 5 is provided with a second stop structure. The limit structure 71 is rotatable relative to the puncture assembly 5 to stop the first stop structure with the second stop structure or disengage the first stop structure from the second stop structure.

In an embodiment, as illustrated in FIG. 15, the limit hole 711 and the puncture assembly 5 both have non-circular structures. The limit hole 711 can rotate together with the limit structure 71 relative to the puncture assembly 5. When the limit hole 711 rotates to a position where the limit hole 711 coincides with the puncture assembly 5, the limit structure 71 is in an unlocked state. In this case, the puncture assembly 5 is no longer stopped by the limit structure 71 and can therefore pass out of the limit hole 711. When the limit structure 71 is rotated until the limit hole 711 is offset from the puncture assembly 5, the limit structure 71 is in a locked state and stops the puncture assembly 5, in such a manner that the puncture assembly 15 cannot pass through the limit hole 711.

In another embodiment, the first stop structure is constructed as a protrusion structure disposed at an inner wall of the limit hole 711. The puncture assembly 5 is correspondingly provided with a groove structure at an outer wall of the puncture assembly 5. When the protrusion structure and the groove structure are rotated until the protrusion structure and the groove structure coincide with each other, the puncture assembly 5 is unlocked. When the protrusion structure and the groove structure are offset from each other, the puncture assembly 5 is locked.

Further, as illustrated in FIG. 14 and FIG. 15, the implant apparatus further includes the cap 6 configured to block the opening of the outer housing 1. The cap 6 cooperates with the limit structure 71 to allow the limit structure 71 to be linked with the cap 6.

As illustrated in FIG. 16, the cap 6 is configured for closing the opening before the implant apparatus is used, and abuts with a bottom wall of the skin fixing base 8 to form a sealed cavity 61, enabling a needle of the sensor 82 and the puncture assembly 5 to be in a closed sterile environment. During use of the implant apparatus, the cap 6 is removed by a rotation. The needle of the sensor 82 and the needle body 51 are exposed in this case, which facilitates the implantation operation for the user. Further, during the removal of the cap 6, the limit structure 71 rotates synchronously, in such a manner that the puncture assembly 5 is unlocked, enabling the puncture assembly 5 to move for the implantation operation. In this way, the cap 6 and the puncture assembly 5 are synchronously unlocked. Only one step is taken to unlock multiple members, which simplifies operation steps, reduces complexity in using the product, and improves the use experience.

Any aspects not described in the present disclosure can be realized by adopting or referencing the related art.

Each embodiment in the specification is described in a progressive manner. The same or similar parts of various embodiments can be referred to each other. Each embodiment focuses on differences from other embodiments.

While some embodiments of the present disclosure have been described above, the present disclosure is not limited to these embodiments. It is conceivable for those skilled in the art that various changes and variations can be made to the present disclosure. Any modifications, equivalent replacements, improvements, or the like made within the spirit and the principle of the present disclosure shall equally fall within the scope of the present disclosure as defined by the claims as attached.

## Claims

1. An implant apparatus for a continuous glucose monitor, the implant apparatus comprising:
a housing assembly comprising an outer housing, the outer housing having an opening at an end of the outer housing;
a driving assembly disposed in the housing assembly, the driving assembly having a locked state in which the driving assembly is fixed relative to the outer housing and a triggered state in which the driving assembly is movable towards the opening; and
a trigger;
wherein the outer housing comprises a guiding channel located at a side wall of the outer housing and extending towards the opening; and
the trigger is movable towards the opening along the guiding channel and has a first position and a second position relative to the outer housing, to enable the driving assembly to switch from the locked state to the triggered state.

2. The implant apparatus according to claim 1, wherein the trigger covers the guiding channel when the trigger is located at the first position or the second position.

3. The implant apparatus according to claim 1, further comprising a cap configured to block the opening and configured to engage with the trigger to restrict a movement of the trigger.

4. The implant apparatus according to claim 1, wherein the trigger surrounds an outer periphery of the housing assembly.

5. The implant apparatus according to claim 1, wherein:
the housing assembly is provided with an engagement wing, the engagement wing is provided with a stop rib;
the trigger comprises a triggering portion extending into the outer housing through the guiding channel; and
the triggering portion is configured to press the engagement wing to displace the stop rib, to enable the driving assembly to switch from the locked state to the triggered state.

6. The implant apparatus according to claim 5, wherein the engagement wing has an inclined engagement surface at an outer side of the engagement wing, the triggering portion being engaged with the inclined engagement surface to force the engagement wing to move towards an interior of the outer housing.

7. The implant apparatus according to claim 5, wherein the engagement wing has a fixed end fixedly connected to the housing assembly, and a free end, the inclined engagement surface and the stop rib being disposed at the free end.

8. The implant apparatus according to claim 5, wherein the driving assembly has an engagement rib,
wherein the driving assembly is in the locked state when the stop rib is in a stop engagement with the engagement rib; and
wherein the driving assembly is in the triggered state when the stop rib is offset from the engagement rib.

9. The implant apparatus according to claim 5, wherein the housing assembly further comprises an inner housing disposed in the outer housing, the engagement wing being disposed at the inner housing.

10. The implant apparatus according to claim 9, wherein:
the outer housing is provided with a snap portion at an inner wall of the outer housing; and
the inner housing is provided with an engagement portion at an outer wall of the inner housing, the snap portion being engaged with and fixed to the engagement portion.

11. The implant apparatus according to claim 1, wherein:
the outer housing comprises an upper housing and a lower housing assembled with each other,
wherein the upper housing has an upper slide channel; and
wherein the lower housing has a lower slide channel, the upper slide channel being engaged with the lower slide channel to form the guiding channel.

12. The implant apparatus according to claim 1, wherein the trigger is provided with a force application portion.

13. The implant apparatus according to claim 1, further comprising a puncture assembly fixed to the driving assembly, wherein:
the driving assembly has a distal position and a proximal position;
the driving assembly is configured to drive the puncture assembly to move relative to the housing assembly from the distal position to the proximal position;
the driving assembly is provided with a limit portion configured to limit a position of the puncture assembly; and
the housing assembly is provided with an unlocking structure, wherein the unlocking structure is configured to release a positional limitation of the limit portion when the driving assembly is moved to the proximal position, to move the puncture assembly away from the opening relative to the driving assembly.

14. The implant apparatus according to claim 13, further comprising a connection base, a skin fixing base, and a sensor, wherein:
the connection base is fixed to the driving assembly, and has a mounting site inside the connection base, the mounting site being configured for mounting of the skin fixing base;
the skin fixing base has a through hole;
the sensor is at least partially received in the puncture assembly;
the sensor has an end located in the skin fixing base and another end extending downwards through the through hole;
the puncture assembly passes through the through hole; and
the connection base is provided with a limit structure configured to at least restrict the puncture assembly from moving out of the through hole.

15. The implant apparatus according to claim 14, further comprising a cap configured to block the opening of the outer housing and cooperating with the limit structure to allow the limit structure to be linked with the cap.

16. The implant apparatus according to claim 13, wherein:
the driving assembly has a slide channel configured to guide needle assistance and/or needle withdrawal of the puncture assembly;
the limit portion comprises a stop protrusion disposed at the slide channel; and
the puncture assembly has an abutment wing configured to abut with the stop protrusion to restrict the puncture assembly from moving away from the opening.

17. The implant apparatus according to claim 16, wherein the unlocking structure comprises an unlocking rib disposed at the housing assembly and configured to abut against the abutment wing to move the abutment wing and disengage the abutment wing from the stop protrusion.
